# EUROPÄISCHE PATENTANMELDUNG

(11) **EP 0 629 618 A1**
(43) Veröffentlichungstag der Anmeldung: **21.12.1994**
(21) Anmeldenummer: 94108444.4
(22) Anmeldetag: 01.06.1994
(51) Int. Cl.: C07D 277/62, C07B 61/00

(54) **Verfahren zur Entschwefelung organischer Mercapto- und/oder Disulfid-Verbindungen**

(30) Priorität: 14.06.1993 DE 4319574
(71) Anmelder: BAYER AG, 51368 Leverkusen (DE)
(72) Erfinder: Hagedorn, Ferdinand, Dr., D-51381 Leverkusen (DE); Fiege, Helmut, Dr., D-51373 Leverkusen (DE); Traenckner, Hans-Joachim, Dr., D-51375 Leverkusen (DE)

(57) **Zusammenfassung**

Schwefelärmere und schwefelfreie organische Verbindungen können aus organischen Mercapto- und/oder Disulfid-Verbindungen in besonders vorteilhafter Weise durch hydrogenolytische Schwefelwasserstoff-Abspaltung hergestellt werden, wenn man die Schwefelwasserstoff-Abspaltung durchführt in Gegenwart eines organischen Lösungsmittels mit einer wäßrigen, nicht oxidierenden, starken Säure und elementarem Eisen, Aluminium und/oder Zink in Gegenwart von katalytischen Mengen Nickel und/oder Kobalt.

## Beschreibung

Die vorliegende Erfindung betrifft ein Verfahren zur Herstellung schwefelärmerer und schwefelfreier organischer Verbindungen aus organischen Mercapto- und/oder Disulfid-Verbindungen durch hydrogenolytische Schwefelwasserstoff-Abspaltung.

Die Entschwefelung von Mercapto- Verbindungen mit Raney-Nickel ist Gegenstand einer Vielzahl von Veröffentlichungen (siehe z.B. Chem. Rev. 62, 347-404 (1962) und Org. Reactions 12, 356-529 (1962)). Hierbei handelt es sich um Methoden, bei denen dem Raney-Nickel anhaftender oder durch basische Hydrolyse von Raney-Legierungen entstehender Wasserstoff das eigentliche Agens ist. Diese Reaktionen werden im allgemeinen im alkalischen bis neutralen Bereich durchgeführt. Deshalb ist ein sehr hoher Raney-Nickel-Überschuß erforderlich.

Bei der Reduktion von 2-Mercaptobenzthiazol mit Eisenpulver in einem Essigsäure-Ethanol-Wasser-Gemisch wurde Benzthiazol mit einer Ausbeute von nur 51 % der Theorie erhalten (siehe Acta, Chem. Scand. 16(4), 1052 (1962)).

Weitere hydrierende Verfahren zur Herstellung von Benthiazol aus 2-Mercaptobenzthiazol (siehe z.B. US-A 2402 642 und CS-A 263891) arbeiten mit Kobalttrisulfid als Katalysator und erfordern hohe Drucke und sicherheitstechnisch nur sehr aufwendig handhabbare Lösungsmittel (z.B. 1,4-Dioxan) wenn man hohe Ausbeuten erzielen will. Die Ausbeuten bei solchen Verfahren liegen im Bereich von 80 bis 99 % der Theorie.

Es wurde nun ein Verfahren zur Herstellung schwefelärmerer und schwefelfreier organischer Verbindungen aus organischen Mercapto- und/oder Disulfid-Verbindungen durch hydrogenolytische Schwefelwasserstoff-Abspaltung gefunden, das dadurch gekennzeichnet ist, daß man die Schwefelwasserstoff-Abspaltung durchführt in Gegenwart eines organischen Lösungsmittels mit einer wäßrigen, nicht oxidierenden, starken Säure und elementaren Eisen, Aluminium und/oder Zink in Gegenwart von katalytischen Mengen Nickel und/oder Kobalt.

In das erfindungsgemäße Verfahren können die verschiedensten organischen Mercapto- und Disulfid-Verbindungen eingesetzt werden. Bevorzugt werden cyclische, stickstoffhaltige Mercapto- und Disulfid-Verbindungen eingesetzt, bei denen das Stickstoffatom oder die Stickstoffatome im Ring oder als Substituenten am Ring angeordnet sein können. Bei den Ringen kann es sich beispielsweise um monocyclische oder bicyclische Systeme handeln, bei denen beispielsweise jeweils 5 oder 6 Atome einen Ring bilden.

Besonders bevorzugte Einsatzmaterialien für das erfindungsgemäße Verfahren sind Mercapto- und Disulfid-Verbindungen von Benzthiazolen und Benzimidazolen, wie 2-Mercapto-benzthiazol, 2-Mercaptobenzimidazol und 2,2'-Bis-(Benzothiazolyl)-disulfid.

Beim erfindungsgemäßen Verfahren werden die Mercapto- bzw. Disulfid-Gruppen aus den Einsatzmaterialien im allgemeinen sehr selektiv entfernt. So erhält man beispielsweise aus Mercaptobenzthiazolen und Bis-(benzothiazolyl)-disulfiden die entsprechenden Benzthiazole, wobei eine weitere Entschwefelung, des Benzthiazols (beispielsweise zu N-Methylanilin) praktisch nicht eintritt. Setzt man in das erfindungsgemäße Verfahren beispielsweise Mercapto- oder Disulfid-Verbindungen von Benzimidazolen ein, so erhalt man die entsprechenden Benzimidazole.

Als organische Lösungsmittel für das erfindungsgemäße Verfahren kommen beispielsweise solche infrage, die im Reaktionsgemisch inert und mit starken wäßrigen Säuren mischbar sind. Geeignet sind beispielsweise aliphatische Alkohole mit 1-6 C-Atomen und Carbonsäuren mit 1 bis 4 C-Atomen. Bevorzugt sind Methanol, Ethanol, Isopropanol und Essigsäure, insbesondere Methanol. Lösungsmittel kann man, bezogen auf 1 Gramm eingesetzter organischer Mercapto- oder Disulfid-Verbindung, beispielsweise in Mengen von 0,5 bis 50 ml einsetzen. Es ist nicht erforderlich, soviel Lösungsmittel zuzufügen, bis eine klare Lösung des Einsatzmaterials vorliegt, da man das erfindungsgemäße Verfahren auch dann durchführen kann, wenn das Einsatzmaterial ganz oder teilweise in suspendierter Form vorliegt.

Als wäßrige, nicht oxidierende, starke Säure kommen insbesondere solche infrage, bei deren Einwirkung sich Eisen, Aluminium und/oder Zink unter Wasserstoffentwicklung auflösen und in denen sich unter Reaktionsbedingungen keine schwerlöslichen Sulfide von Eisen, Aluminium und/oder Zink bilden. Bevorzugt sind wäßrige Mineralsäuren, insbesondere wäßrige Salzsäure und wäßrige Schwefelsäure, z.B. 5-40 gew.-%ige wäßrige Salzsäure und 5-40 gew.-%ige wäßrige Schwefelsäure. Besonders bevorzugt ist 25-40 gew.-%ige wäßrige Salzsäure.

Die Säure wird zweckmäßigerweise wenigstens in der Menge eingesetzt, die theoretisch erforderlich ist, um mit dem eingesetzten elementaren Eisen, Aluminium und/ oder Zink soviel Wasserstoff zu entwickeln, wie theoretisch erforderlich ist, um die im Einsatzprodukt vorhandenen Mercapto- und Disulfidgruppen in Schwefelwasserstoff zu überführen. Vorzugsweise setzt man einen Überschuß der Säure ein, beispielsweise bis zur doppelten der theoretisch erforderlichen Menge.

Eisen, Aluminium und/oder Zink werden in elementarer und vorzugsweise in feinverteiler Form eingesetzt. Bevorzugt sind Eisen und Aluminium, insbesondere Eisenfeilspäne, Eisenpulver, verdüstes Eisen und/oder Aluminiumpulver.

Bezogen auf 1 Mol Mercapto- bzw. Disulfid-Verbindung kann man beispielsweise 1-12 Mol Eisen, Aluminium und/oder Zink einsetzen Bezogen auf 1 Mol eingesetzte Mercaptoverbindung werden vorzugsweise 1-2 Mol, bezogen auf 1 Mol eingesetzte Disulfid-Verbindung vorzugsweise 2-4 Mol Eisen, Aluminium und/oder Zink eingesetzt. Größere Mengen der Metalle können besonders dann von Vorteil sein, wenn die Metalle nicht in feinverteilter Form vorliegen.

Es ist vorteilhaft, während der Durchführung des erfindungsgemäßen Verfahrens für einen guten Kontakt zwischen dem sich entwickelnden Wasserstoff und den eingesetzten Mercapto- oder Disulfid-Verbindungen zu sorgen. Hierzu kann man z.B. an sich bekannte Rühr- oder Blasensäulentechniken anwenden.

Es ist ein wesentliches Merkmal des erfindungsgemäßen Verfahrens, daß man es in Gegenwart von katalytischen Mengen Nickel und/oder Kobalt durchführt. Nickel und/oder Kobalt kann dabei in metallischer Form oder in Form von Salzen zugefügt werden. Bevorzugt wird elementares Nickel oder elementares Kobalt als Raney-Nickel oder Raney-Kobalt eingesetzt. Besonders bevorzugt sind dabei sogenannte Altkatalysatoren, d.h. Raney-Nickel, Raney-Kobalt oder Raney-Nickel-Kobalt, das bereits in anderen Verfahren, z.B. in katalytischen Hydrierungen mit elementarem Wasserstoff als Katalysator verwendet worden ist. Insbesondere Raney-Nickel kann auch in Form von Raney-Nickel-Eisen zugesetzt werden. Salzförmiges Nickel und/oder Kobalt kann beispielsweise in Form der Chloride eingesetzt werden.

Bezogen auf 1 Mol eingesetztes elementares Eisen, Aluminium und/oder Zink kann man beispielsweise 0,01-0,5 Mol Nickel und/oder Kobalt in elementarer Form oder in Salzform einsetzen. Vorzugsweise liegt diese Menge bei 0,01-0,3 Mol.

Das erfindungsgemäße Verfahren kann man beispielsweise bei Temperaturen im Bereich von 0-120°C durchführen. Bevorzugt sind Temperaturen im Bereich von 40-100°C. Besonders bevorzugt wird mit einen, am Rückfluß siedenden Lösungsmittel gearbeitet.

Das erfindungsgemäße Verfahren kann bei Normaldruck, erhöhtem oder erniedrigtem Druck durchgerührt werden. Vorzugsweise arbeitet min bei Normaldruck. Das Arbeiten bei erniedrigtem oder erhöhtem Druck, z.B. im Bereich von 0.1-5 bar, kann von Interesse sein, wenn man bei am Rückfluß siedendem Lösungsmittel arbeiten will, jedoch bei einer anderen Temperatur als dem Siedepunkt des jeweiligen Lösungsmittels bei Normaldruck.

Das erfindungsgemäße Verfahren kann man beispielsweise so durchführen, daß man die Mercapto- bzw. Disulfid-Verbindung zusammen mit dem Lösungsmittel und elementarem Eisen, Aluminium und/oder Zink, sowie Nickel und/oder Kobalt in metallischer oder in Salzform vorlegt, dann auf die gewünschte Reaktionstemperatur erhitzt und danach die Säure unter kräftigem Rühren zufließen läßt. Der sich entwickelnde Schwefelwasserstoff kann in an sich bekannter Weise aufgefangen und entsorgt werden, beispielsweise durch eine Wäsche mit wäßriger Natronlauge oder durch Einspeisung in einen Claus-Prozeß zur Herstellung von elementarem Schwefel.

Nach Beendigung der Säurezugabe ist es vorteilhaft mindestens so lange bei Reaktionstemperatur nachzurühren, bis die Wasserstoffentwicklung zu Ende gekommen ist. Sofern noch überschüssiges elementares Eisen, Aluminium und/oder Zink vorhanden ist, scheidet sich daran im Reaktionsgemisch in gelöster Form befindliches Nickel und/oder Kobalt ab. Die Aufarbeitung des Reaktionsgemisches kann so erfolgen, daß man zunächst die flüssige Phase von evtl. vorhandenem Metallrückstand abtrennt, das Lösungsmittel durch Destillation zurückgewinnt und das erhaltene Produkt durch Wasserdampfdestillation oder Extraktion mit einem geeigneten Lösungsmittel isoliert. Den abgetrennten Metallrückstand kann man wiederverwenden. Da er praktisch das gesamte eingesetzte Nickel und/oder Kobalt enthält, muß dieses nicht ständig von außen zugeführt werden.

Diese detailliert beschriebene Ausführungsform der vorliegenden Erfindung kann in vielerlei Weise variiert werden. Man kann das erfindungsgemäße Verfahren auch beispielsweise kontinuierlich oder diskontinuierlich durchführen.

Das erfindungsgemäße Verfahren liefert im allgemeinen schwefelärmere und/oder schwefelfreie Produkte aus organischen Mercapto- und Disulfid-Verbindungen in Ausbeuten von über 90 % der Theorie.

Das erfindungsgemäße Verfahren besitzt gegenüber bisher bekannten Hydriermethoden die wesentlichen Vorteile, daß es bei Normaldruck ausgeübt werden kann und daß dabei nur katalytische Mengen Nickel und/oder Kobalt benötigt werden. Ein weiterer Vorteil besteht in der schonenden Reaktionstemperatur, die man beispielsweise im Bereich zwischen Raumtemperatur und der Siedetemperatur des verwendeten Reaktionsmediums wählen kann. Überraschend sind die trotz der milden Reaktionsbedingungen realisierbaren hohen Ausbeuten und Selektivitäten. Der größte Vorteil der vorliegenden Erfindung ist darin zu sehen, daß im Gegensatz zu bisher nur katalytische Mengen Nickel und/oder Kobalt benötigt werden, was aus ökologischen Gründen sehr wünschenswert ist. Trotzdem liegen die Ausbeuten hoch und z.B. deutlich günstiger als bei der Arbeitsweise nur mit Eisen.

Die folgenden experimentellen Beispiele erläutern das erfindungsgemäße Verfahren, ohne es auf sie zu beschränken.

### Beispiel 1:

87,9 g 2-Mercaptobenzthiazol, 111,7 g Eisenspäne und 9,5 g Nickelchlorid (NiCl₂ 6H₂O) wurden in 250 ml Methanol suspendiert. Unter Rühren wurde das Gemisch bis zum Sieden am Rückfluß (ca. 70°C) erhitzt und dann innerhalb von 2 Stunden 165 ml konzentrierte wäßrige Salzsäure zugetropft. Der sich entwickelnde Schwefelwasserstoff wurde in einer nachgeschalteten Natronlauge-Wäsche aufgefangen. Das Gemisch wurde anschließend 1 Stunde bei 70°C nachgerührt, danach abgekühlt, der ungelöste Metall-Rückstand abgetrennt und die erhaltene klare Lösung mittels HPLC-Analyse untersucht. Es ergab sich eine Ausbeute von 64,7 g Benzthiazol, entsprechend 96 % der Theorie.

### Beispiel 2:

16,7 g 2-Mercaptobenzthiazol, 13,6 g Zinkstaub und 11,8 g Nickelchlorid (NiCl₂6H₂O) wurden in 250 ml Methanol suspendiert und auf 70°C unter Rühren erhitzt. Unter Kochen am Rückfluß wurden bei 70°C 50 ml konzentrierte wäßrige Salzsäure innerhalb von 2 Stunden zugetropft, wobei sich bildender Schwefelwasserstoff gasförmig in einer nachgeschalteten Natronlauge-Wäsche aufgefangen wurde. Nach einer Nachrührzeit von 30 Minuten wurde vom ungelösten Metallrückstand abgetrennt und die erhaltene Lösung einer HPLC-Analyse unterworfen. Es ergab sich ein Gehalt von 12,5 g Benzthiazol, entsprechend einer Ausbeute von 90.3 % der Theorie.

### Beispiel 3:

16,6 g Bis-(benzothiazolyl)-disulfid, 22,4 g Eisenspäne und 11,8 g Nickelchlorid (NiCl₂6H₂O) wurden in 250 ml Methanol suspendiert. Unter Rühren wurde das Gemisch auf 70°C erhitzt, und es wurden unter Sieden am Rückfluß innerhalb von 2 Stunden 50 ml konzentrierte wäßrige Salzsäure zugefügt. Der sich entwickelnde Schwefelwasserstoff wurde in einer Natronlauge-Wäsche aufgefangen. Das Reaktionsgemisch wurde 30 Minuten bei 70°C nachgenührt. Nach dem Abkühlen wurde das Gemisch abgesaugt und 11,5 g metallischer Rückstand erhalten. Die HPLC-Analyse der abgetrennten Lösung ergab das Vorhandensein von 12,7 g Benzthiazol, entsprechend 94 % der Theorie.

### Beispiel 4:

15,0 g 2-Mercaptobenzimidazol, 22,4 g Eisenspäne und 11,8 g Nickelchlorid (NiCl₂6H₂O) wurden in 250 ml Methanol suspendiert und unter Rühren bis zum Rückflußsieden des Methanols erhitzt. Innerhalb von 2 Stunden wurden dann 50 ml konzentrierte wäßrige Salzsäure zugetropft und der sich entwickelnde Schwefelwasserstoff in einer Natronlauge-Wäsche aufgefangen. Das Gemisch wurde 30 Minuten bei 70°C nachgerührt, auf Raumtemperatur abgekühlt und durch Absaugen vom metallischen Rückstand abgetrennt. Die hinterbleibende Lösung enthielt gemäß HPLC-Analyse 10,9 g Benzimidazol, entsprechend 91,9 % der Theorie.

### Beispiel 5:

87,9 g Mercaptobenzthiazol (95 %ig), 111,7 g Eisenspäne und 3 g gebrauchter Raney-Nickel-Katalysator wurden in einem Rührkolben vorgelegt und in 250 ml Methanol unter Rühren auf Rückflußtemperatur erhitzt. Bei 70°C wurden innerhalb von 2 Stunden 165 ml konzentrierte wäßrige Salzsäure zugetropft und der sich entwickelnde Schwefelwasserstoff in einer wäßrigen Natronlauge-Lösung aufgefangen. Das Gemisch wurde 1 Stunde bei 70°C nachgerührt, dann auf Raumtemperatur abgekühlt und abgesaugt. Das Filtrat und das aus Methanol und Wasser bestehende Nachwaschmittel wurden vereinigt und durch eine HPLC-Analyse untersucht. Es lagen 65,9 g Benzthiazol vor, entsprechend einer Ausbeute von 97,6 % der Theorie.

### Beispiel 6:

87,9 g 2-Mercaptobenzthiazol (95 %ig), 111,7 g Eisenspäne, 11,8 g Nickelchlorid (NiCl₂ x 6 H₂O) und 250 ml Methanol wurden in einem Rührkolben vorgelegt und unter Rühren auf Rückflußtemperatur erhitzt. Bei 70°C wurden innerhalb 2 Stunden 165 ml konz. wäßrige Salzsäure zugetropft und der sich entwickelnde Schwefelwasserstoff in waßriger Natronlauge aufgefangen. Das Gemisch wurde 1 Stunde bei 70°C nachgerührt, anschließend mit 200 ml Wasser verdünnt. 200 ml Methanol wurden abdestilliert. Das zurückbleibende Gemisch wurde mit wäßriger Natronlauge auf pH 5,6 eingestellt und mit Wasserdampf destilliert. Das Wasserdampfdestillat wurde mit Toluol extrahiert. Nach Abdestillieren des Lösungsmittels fielen 66,3 g rohes, farbloses Benzthiazol an, 93,7 %ig (GC) entsprechend 92 % Ausbeute d. Th. mit einem Rest-Toluol-Gehalt von 5,3 %.

## Patentansprüche

1. Verfahren zur Herstellung schwefelärmerer und schwefelfreier organischer Verbindungen aus organischen Mercapto- und/oder Disulfid-Verbindungen durch hydrogenolytische Schwefelwasserstoff-Abspaltung, dadurch gekennzeichnet, daß man die Schwefelwasserstoff-Abspaltung durchführt in Gegenwart eines organischen Lösungsmittels mit einer wäßrigen, nicht oxidierenden, starken Säure und elementarem Eisen, Aluminium und/oder Zink in Gegenwart von katalytischen Mengen Nickel und/oder Kobalt.

2. Verfahren nach Anspruch 1, dadurch gekennzeichnet, daß man cyclische, stickstoffhaltige Mercapto- und/oder Disulfid-Verbindungen einsetzt.

3. Verfahren nach Ansprüchen 1 und 2, dadurch gekennzeichnet, daß man als organisches Lösungsmittel aliphatische Alkohole mit 1-6 C-Atomen oder Carbonsäure mit 1-4 C-Atomen einsetzt.

4. Verfahren nach Ansprüchen 1-3, dadurch gekennzeichnet, daß man das organische Lösungsmittel in Mengen von 0,5-50 ml, bezogen auf 1 g eingesetzter organischer Mercapto- und/oder Disulfid-Verbindung, einsetzt.

5. Verfahren nach Ansprüchen 1-4, dadurch gekennzeichnet, daß man als wäßrige, nicht oxidierende, starke Säure wäßrige Salzsäure oder wäßrige Schwefelsäure einsetzt.

6. Verfahren nach Ansprüchen 1-5, dadurch gekennzeichnet, daß man die wäßrige, nicht oxidierende, starke Säure wenigstens in der Menge einsetzt, die theoretisch erforderlich ist, um mit dem eingesetzten elementaren Eisen, Aluminium und/oder Zink soviel Wasserstoff zu entwickeln, wie theoretisch erforderlich ist, um die im Einsatzprodukt vorhandenen Mercapto- und Disulfid-Gruppen in Schwefelwasserstoff zu überführen.

7. Verfahren nach Ansprüchen 1-6, dadurch gekennzeichnet, daß man elementares Eisen, Aluminium und/oder Zink in feinverteilter Form einsetzt.

8. Verfahren nach Ansprüchen 1-7, dadurch gekennzeichnet, daß man auf 1 Mol Mercapto- bzw. Disulfid-Verbindung 1-12 Mol elementares Eisen, Aluminium und/oder Zink einsetzt.

9. Verfahren nach Ansprüchen 1-8, dadurch gekennzeichnet, daß man Nickel und/oder Kobalt in metallischer Form oder in Form von Salzen und in Mengen von 0,01-0,5 Mol Nickel und/oder Kobalt pro Mol eingesetztes elementares Eisen, Aluminium und/oder Zink einsetzt.

10. Verfahren nach Ansprüchen 1-9, dadurch gekennzeichnet, daß man es bei Temperaturen im Bereich von 0-120°C durchführt.
